(19) Europäisches Patentamt
European Patent Office
Office européen des brevets

(11) Veröffentlichungsnummer: **0 310 558**
**A2**

(12) # EUROPÄISCHE PATENTANMELDUNG

(21) Anmeldenummer: **88810656.4**

(22) Anmeldetag: **26.09.88**

(51) Int. Cl.4: **C 07 D 405/04**
C 07 D 207/34, A 01 N 43/36

(30) Priorität: **02.10.87 CH 3857/87**

(43) Veröffentlichungstag der Anmeldung:
**05.04.89 Patentblatt 89/14**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Ackermann, Peter, Dr.**
**Hangelimattweg 113**
**CH-4148 Pfeffingen (CH)**

**Ehrenfreund, Josef, Dr.**
**Amselstrasse 11**
**CH-4123 Allschwil (CH)**

**Nyfeler, Robert, Dr.**
**Bärenfelserstrasse 8**
**CH-4057 Basel (CH)**

**Martin, Pierre, Dr.**
**Meisenweg 38**
**CH-4310 Rheinfelden (CH)**

(54) **Mikrobizide Mittel.**

(57) Neue Verbindungen der Formel

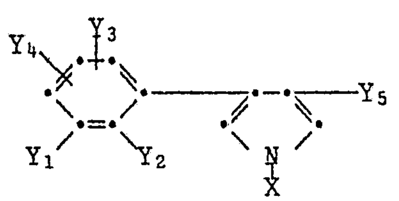

(I),

worin einer der beiden Substituenten $Y_1$ oder $Y_2$ eine $C_1$-$C_3$-Haloalkoxy-Gruppe und der andere Wasserstoff, Halogen, $C_1$-$C_3$-Alkoxy oder eine $C_1$-$C_3$-Haloalkoxy-Gruppe bedeutet, oder worin $Y_1$ und $Y_2$ zusammen mit den beiden Kohlenstoffatomen des Phenylrings einen unsubstituierten oder $C_1$-$C_3$-Alkyl-, $C_1$-$C_3$-Haloalkyl- oder halogensubstituierten 5-Ring-Heterocyclus mit einem oder zwei Sauerstoffatomen oder einen halogensubstituierten 6-Ring-Heterocyclus mit zwei Sauerstoffatomen,
$Y_3$ und $Y_4$ unabhängig voneinander Wasserstoff, Halogen, $C_1$-$C_6$-Alkyl, $C_1$-$C_6$-Alkenyl, $NO_2$, $NH_2$, $CF_3$, CN oder $C_1$-$C_4$-Aliphatoxy bedeuten, und
$Y_5$ für Cl, $CF_3$ oder CN steht, während
X eine im Anspruch 1 angegebenen Bedeutung hat
Die Verbindungen der Formel I haben mikrobizide Wirkung.

EP 0 310 558 A2

**Beschreibung**

## Mikrobizide Mittel

Die vorliegende Erfindung betrifft neue substituierte Phenylpyrrole, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

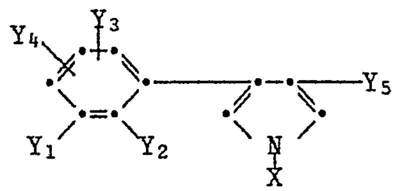 (I),

worin einer der beiden Substituenten $Y_1$ oder $Y_2$ eine $C_1$-$C_3$-Haloalkoxy-Gruppe und der andere Wasserstoff, Halogen, $C_1$-$C_3$-Alkoxy oder eine $C_1$-$C_3$-Haloalkoxy-Gruppe bedeutet, oder worin $Y_1$ und $Y_2$ zusammen mit den beiden Kohlenstoffatomen des Phenylrings einen unsubstituierten oder $C_1$-$C_3$-Alkyl-, $C_1$-$C_3$-Haloalkyl- oder halogensubstituierten 5-Ring-Heterocyclus mit einem oder zwei Sauerstoffatomen oder einen halogensubstituierten 6-Ring-Heterocyclus mit zwei Sauerstoffatomen,
$Y_3$ und $Y_4$ unabhängig voneinander Wasserstoff, Halogen, $C_1$-$C_6$-Alkyl, $C_1$-$C_6$-Alkenyl, $NO_2$, $NH_2$, $CF_3$, CN oder $C_1$-$C_4$-Aliphatoxy bedeuten, und
$Y_5$ für Cl, $CF_3$ oder CN steht, während
X eine der folgenden Bedeutungen hat:

A) Wasserstoff,

B) einen Sulfonsäurerest $SO_2$-R, wobei R unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy oder $C_3$-$C_6$-Cycloalkyl, unsubstituiertes oder durch Halogen, $NO_2$, $C_1$-$C_4$-Alkyl oder $C_1$-$C_4$-Alkoxy substituiertes Phenyl darstellt;

C) Wasserstoff oder den Acylrest einer Carbonsäure;

D) einen Rest S-$R_6$, worin $R_6$ $C_1$-$C_3$-Haloalkyl bedeutet;

E) einen Rest

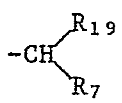

wobei $R_7$ für Wasserstoff oder $C_1$-$C_8$-Haloalkyl $R_{19}$ für Hydroxy, Halogen oder $OC(O)R_8$ stehen, worin $R_8$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

F) einen Rest $CH_2$-Z, wobei Z für eine der Gruppen

a) 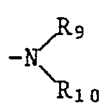 oder b) 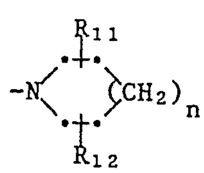 oder c) 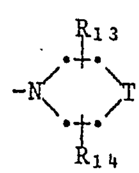

steht, worin $R_9$ und $R_{10}$ unabhängig voneinander Wasserstoff, unsubstituiertes oder durch Cyano, $C_1$-$C_6$-Alkoxycarbonyl substitutiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_9$ oder $R_{10}$ Wasserstoff sein kann; $R_{11}$ und $R_{12}$ unabhängig voneinander für Wasserstoff, $C_1$-$C_6$-Alkyl, $C_1$-$C_6$-Alkoxycarbonyl darstellen oder beide zusammen einen ankondensierten aromatischen Ring bilden; $R_{13}$ und $R_{14}$ unabhängig voneinander für

Wasserstoff, $C_1$-$C_6$-Alkyl oder $C_1$-$C_6$-Alkoxycarbonyl stehen; und T Sauerstoff, Schwefel, >C=O oder >N-$R_{15}$ bedeutet;
worin $R_{15}$ Wasserstoff, $C_1$-$C_6$-Alkyl, Formyl; $C_1$-$C_6$-Alkanoyl oder $C_1$-$C_6$-Alkoxycarbonyl darstellt; und n für eine der Zahlen 0 oder 1 steht;

G) einen Silylrest -Si($R_{16}$)($R_{17}$)($R_{18}$), wobei $R_{16}$, $R_{17}$ und $R_{18}$ unabhängig voneinander für $C_1$-$C_4$-Alkyl, Benzyl oder Phenyl stehen; wobei X stets einen Sulfonsäurerest oder einen Silylrest bedeutet, wenn gleichzeitig $Y_1 = Y_2 = F$ und $Y_3 = Y_4 = H$ und $Y_5$ = Cyano oder gleichzeitig $Y_1$ und $Y_2$ zusammen -O-$CF_2$-O- und $Y_3 = Y_4 = H$ und $Y_5$ = Cyano sind.

Unter dem Begriff Alkyl selbst oder als Bestandteil eines anderen Substituenten sind je nach der Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw.. Haloalkyl steht für einen einfach bis perhalogenierten Alkylsubstituierten, wie z.,B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw. Unter Halogen soll hier und im folgenden Fluor, Chlor, Brom oder Jod, vorzugsweise Fluor oder Chlor verstanden werden. Unter Alyphatoxy kann neben Alkoxy auch Alkenyloxy, wie z.B. Propenyl-1-oxy, Allyloxy, Butenyl-1-oxy, Butenyl-2-oxy, Butenyl-3-oxy oder Alkinyloxy, wie z.B. Propargyloxy, Butinyl-2-oxy oder Butinyl-3-oxy verstanden werden.

Haloalkoxy steht je nach Zahl der Kohlenstoffatome für eine geradkettige einfach- bis perhalogenierte, über Sauerstoff gebundene Alkylgruppe, die gleiche oder unterschiedliche Halogenatome aufweist.

Beispiele derartiger halogenierter Gruppen sind:
$OCH_2F$, -$OCHF_2$, -$OCF_3$, -$OCH_2Cl$, -$OCCl_3$, -OCHFCl, -$OCClF_2$, -$OCF_2CHF_2$, -$OCF_2CFCl_2$, -$OCF_2CHFCl$, -$OCF_2CF_2Cl$, -$OCF_2CCl_3$,m -$OCF_2CHCl_2$, -$OCH_2CF_3$, -$OCH_2CH_2CL$, -$OCH_2CH_2F$, -$OCF_2CHFCF_3$, -$OCBrF_2$, -$OCH_2$-$CCl_3$ oder -$OC_2F_5$. Bevorzugte Gruppen sind -$OCHF_2$, -$OCF_2$-$CHF_2$, -$OCF_2$-$CFCl_2$, -$OCF_2$-CHFCl oder -$OCF_3$.

Die halogensubstituierten 5-Ring- resp. 6-Ring-Heterocyclen können einfach- bis perhalogeniert sein, wobei die Kohlenstoffatome durch gleiche oder unterschiedliche Halogenatome substituiert sind. Beispiele solcher halogenierter Ringglieder

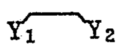

sind:
-$CF_2$-$CF_2$-O-, -O-$CF_2$-$CF_2$-, -O-$CCl_2$-$CCl_2$-, -$CF_2$-O-$CF_2$-, -O-$CF_2$-O-$CF_2$, -$CF_2$-O-$CF_2$-O-, -O-$CF_2$-$CH_2$-O-, O-$CF_2$-CHF-O-, -O-$CCl_2$-$CCl_2$-O-, -$CF_2$-O-$CFCF_3$-O-, -O-$CF_2$-CFCl-O-.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Aufgrund ihrer ausgeprägten mikrobiziden Wirkung sind die Verbindungen der Formel I′

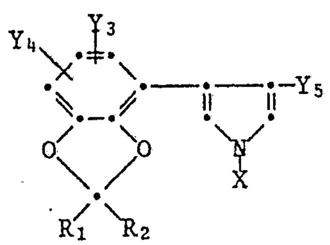

(I'),

bevorzugt, worin
$R_1$ und $R_2$ unabhängig voneinander Wasserstoff, Fluor, Chlor, $C_1$-$C_3$-Alkyl, $C_1$-$C_3$-Haloalkyl bedeuten, während X eine der folgenden Bedeutungen hat:

A) Wasserstoff,

B) einen Sulfonsäurerest $SO_2$-R, wobei R unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy oder $C_3$-$C_6$-Cycloalkyl darstellt;

C) einen Carbosäurerest CO-R, wobei R die unter B) genannte Bedeutung hat, ein Tetrahydrofuryl(2)-Rest ist oder einen unsubstituierten oder durch Halogen, $NO_2$, $C_1$-$C_4$-Alkyl oder $C_1$-$C_4$-Alkoxy

substituierten Phenyl-oder Benzylrest darstellt.
D) einen Rest S-$R_6$, worin $R_6$ $C_1$-$C_3$-Haloalkyl bedeutet;
E) einen Rest

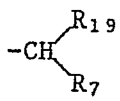

wobei $R_7$ für Wasserstoff oder $C_1$-$C_8$-Haloalkyl $R_{19}$ für Hydroxy, Halogen oder OC(O)$R_8$ stehen, worin $R_8$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;
F) einen Rest $CH_2$-Z, wobei Z für einen der Gruppen

a) 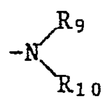 oder b) 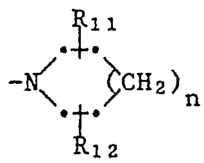 oder c) 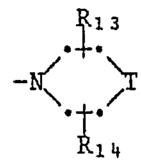

steht, worin $R_9$ und $R_{10}$ unabhängig voneinander Waserstoff, unsubstituiertes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_9$ oder $R_{10}$ Wasserstoff sein kann; $R_{11}$ und $R_{12}$ unabhängig voneinander für Wasserstoff, $C_1$-$C_6$-Alkyl, $C_1$-$C_6$-Alkoxycarbonyl darstellen oder beide zusammen einen ankondensierten aromatischen Ring bilden; $R_{13}$ und $R_{14}$ unabhängig voneinander für Wasserstoff, $C_1$-$C_6$-Alkyl oder $C_1$-$C_6$-Alkoxycarbonyl stehen; und T Sauerstoff, Schwefel, >C=O oder >N-$R_{15}$ bedeutet;
worin $R_{15}$ Wasserstoff, $C_1$-$C_6$-Alkyl, Formyl; $C_1$-$C_6$-Alkanoyl oder $C_1$-$C_6$-Alkoxycarbonyl darstellt; und n für eine der Zahlen 0 oder 1 steht;
G) einen Silylrest -Si($R_{16}$)($R_{17}$)($R_{18}$), wobei $R_{16}$, $R_{17}$ und $R_{18}$ unabhängig voneinander für $C_1$-$C_4$-Alkyl, Benzyl oder Phenyl stehen; wobei X stets einen Sulfonsäurerest oder einen Silylrest bedeutet, wenn gleichzeitig $R_1 = R_2 = F$ und $Y_3 = Y_4 = H$ und $Y_5$ = Cyano sind.

Besonders bevorzugt von dieser Gruppe sind jene Verbindungen, bei welchen
$R_1$ Wasserstoff, Fluor, Chlor, $C_1$-$C_3$-Alkyl, $C_1$-$C_3$-Haloalkyl,
$R_2$ Wasserstoff, Chlor, $C_1$-$C_3$-Alkyl, $C_1$-$C_3$-Haloalkyl, und
$Y_3$ und $Y_4$ unabhängig voneinander Wasserstoff, Halogen, $C_1$-$C_4$-Alkyl, $NO_2$, $NH_2$, $CF_3$, oder $C_1$-$C_4$-Aliphatoxy bedeuten,
$Y_5$ für Cl, $CF_3$ oder CN steht, während
X eine der folgenden Bedeutungen hat:
A) Wasserstoff;
B) einen Sulfonsäurerest $SO_2$-R, wobei R $C_1$-$C_4$-Alky, Phenyl, Chlorphenyl oder Tolyl ist;
C) einen Carbosäurerest CO-R, wobei R $C_1$-$C_6$-Alkyl, $C_1$-$C_4$-Haloalkyl oder durch Halogen oder Methyl substituiertes Phenyl oder Benzyl oder unsubstituiertes Phenyl oder Benzyl bedeutet;
D) einen Rest S-$R_6$, worin $R_6$ $C_1$-$C_3$-Haloalkyl ist;
E) einen Rest

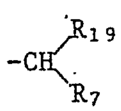

worin $R_7$ für Wasserstoff oder $C_1$-$C_8$-Haloalkyl $R_{19}$ für Hydroxy, Halogen oder O-CO-$R_8$ bedeuten, mit $R_8$ $C_1$-$C_6$-Alkyl oder $C_1$-$C_4$-Haloalkyl;
F) einen Rest $CH_2$-Z, worin z -NH$R_{10}$ oder -N($R_9$)($R_{10}$) darstellt, mit $R_9$ und $R_{10}$ = $C_1$-$C_6$-Alkyl;
G) einen Silylrest -Si($R_{16}$($R_{17}$($R_{18}$), worin $R_{16}$, und $R_{17}$ unabhängig voneinander für $C_1$-$C_4$-Alkyl stehen und $R_{18}$ $C_1$-$C_4$-Alkyl, Benzyl oder Phenyl bedeutet.

Von diesen Verbindungen sind jene insbesondre bevorzugt bei welchen
$R_1$ Fluor, Chlor, Methyl, Ethyl, $CF_3$,
$R_2$ Wasserstoff, Chlor, $C_1$-$C_3$-Alkyl, $CF_3$ und

$Y_3$ und $Y_4$ unabhängig voneinander Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeuten.

Von dieser Gruppe von Verbindungen sind jene hervorzuheben, bei welchen X Wasserstoff bedeutet.

Aus der zuletzt erwähnten Gruppe sind jene von Interesse, bei welchen Wasserstoff ist und $Y_3$ Wasserstoff ist und $Y_4$ in meta-Stellung zum Pyrrolsubstituenten steht und Wasserstoff Halogen, $NO_2$, $NH_2$, $CH_3$, iso$C_3H_7$ oder tert.$C_4H_9$ bedeutet.

Aus dieser Gruppe stechen jene Verbindungen hervor, bei welchen $R_1$ Fluor, Chlor, $CF_3$ oder Methyl und $R_2$ Wasserstoff, Chlor, $CF_3$, Methyl oder Ethyl bedeuten und $Y_5$ für CN oder $CF_3$ steht.

Von den zuletzt definierten Verbindungen sind jene bedeutend, bei welchen $Y_5$ Cyano bedeutet.

Von den Verbindungen der Formel I′ sind auch jene bevorzugt, bei welchen $R_1 = R_2 =$ Fluor ist, $Y_3$ Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeutet, $Y_4$ Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeutet und $Y_5$ Cyano darstellt und
X eine der folgenden Bedeutungen hat:

A) Wasserstoff;

B) einen Sulfonsäurerest $SO_2$-R, wobei R $C_1$-$C_4$-Alkyl, Phenyl, Chlorphenyl oder Tolyl ist;

C) einen Carbosäurerest CO-R, wobei R $C_1$-$C_6$-Alkyl, $C_1$-$C_4$-Haloalkyl oder durch Halogen oder Methyl substituiertes Phenyl oder Benzyl oder unsubstituiertes Phenyl oder Benzyl bedeutet;

D) einen Rest S-$R_6$, worin $R_6$ $C_1$-$C_3$-Haloalkyl ist;

E) einen Rest

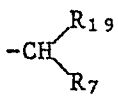

worin $R_7$ für Wasserstoff oder $C_1$-$C_8$-Haloalkyl, $R_{19}$ für Hydroxy, Halogen oder O-CO-$R_8$ bedeuten, mit $R_8$ $C_1$-$C_6$-Alkyl oder $C_1$-$C_4$-Haloalkyl;

F) einen Rest $CH_2$-Z, worin Z -NH$R_{10}$ oder -N($R_9$,)($R_{10}$) darstellt, mit $R_9$ und $R_{10} = C_1$-$C_6$-Alkyl;

G) einen Silylrest -Si)($R_{16}$)($R_{17}$)($R_{18}$), worin $R_{16}$, und $R_{17}$ unabhängig voneinander für $C_1$-$C_4$-Alkyl stehen und $R_{18}$ $C_1$-$C_4$-Alkyl, Benzyl oder Phenyl bedeutet.

Von der zuletzt erwähnten Gruppe sind jene Verbindungen insbesondere bevorzugt bei welchen $Y_3$ Wasserstoff ist und $Y_4$ in meta-Stellung zum Pyrrolsubstituenten steht und Halogen, $NO_2$, $NH_2$, $CH_3$, iso$C_3H_7$ oder tert.$C_4H_9$ bedeutet.

Von den Verbindungen der Formel I′ sind schliesslich Verbindungen bevorzugt bei welchen $Y_3$ und $Y_4$ Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeuten und $Y_5$ Chlor darstellt, während X Wasserstoff ist.

Von der zuletzt erwähnten Gruppe sind jene Verbindungen insbesondere bevorzugt bei welchen $Y_3$ Wasserstoff ist und $Y_4$ in meta-Stellung zum Pyrrolsubstituenten steht und Wasserstoff, Halogen, $NO_2$, $NH_2$, $CH_3$, iso$C_3H_7$ oder tert.$C_4H_9$ bedeutet.

Von den Verbindungen der Formel I sind wegen ihrer markanten mikrobiziden Wirkung auch die Verbindungen der Formel I″

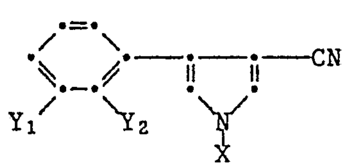

(I″)

bevorzugt, worin einer der beiden Substituenten $Y_1$ oder $Y_2$ eine $C_1$-$C_3$-Haloalkoxy-Gruppe und der andere Wasserstoff, Halogen, $C_1$-$C_3$-Alkoxy oder eine $C_1$-$C_3$-Haloalkoxy-Gruppe bedeutet, oder worin $Y_1$ und $Y_2$ zusammen mit den beiden Kohlenstoffatomen des Phenylrings einen halogensubstituierten 5-Ring-Heterocyclus mit einem Sauerstoffatom oder einen halogensubstituierten 6-Ring-heterocyclus mit 2 Sauerstoffatomen bedeuten.

Von dieser Gruppe sind diejenigen Verbindungen bevorzugt, bei welchen X für Wasserstoff oder den Acylrest einer gegebenenfalls substituierten $C_1$-$C_8$ aliphatischen oder $C_1$-$C_8$ araliphatischen Carbonsäure oder für einen gegebenenfalls substituierten Benzoylrest steht.

Weiter bevorzugt sind Verbindungen, bei welchen der Benzoylrest X durch Halogen, Alkyl, Haloalkyl, Alkoxy oder Haloalkoxy substituiert ist.

Die Zahl der Halogenatome der $C_1$-$C_3$-Haloalkoxygruppen und der heterocyclischen Ringe beträgt 1 bis n, wenn n die Zahl der maximal möglichen Substitutionen in dem betreffenden Rest bedeutet.

Besonders bevorzugte Halogensubstituenten in den Resten $Y_1$ und $Y_2$ sind Fluor- und Chloratome.

Von grossem Interesse sind Verbindungen, in welchen $Y_1$ und $Y_2$ nur durch Fluoratome substituiert sind.

Eine weitere interessante Untergruppe bilden Verbindungen der Formel I, worin sowohl $Y_1$ als auch $Y_2$ eine Haloalkoxy-Gruppe bedeutet.

Wiederum eine interessante Gruppe bilden Verbindungen, bei welchen $Y_1$ oder $Y_2$ für einen Haloalkoxy-Gruppe steht.

Von besonderem Interesse sind diejenigen Verbindungen, bei welchen die Reste $Y_1$ und $Y_2$ entweder einen 5-Ring mit einem Sauerstoffatom oder aber einen 6-Ring mit zwei Sauerstoffatomen bilden.

Besonders interessante Vertreter solcher Ringglieder sind:

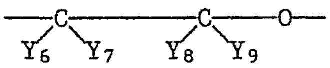 , 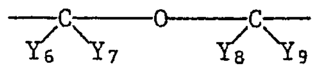 ,

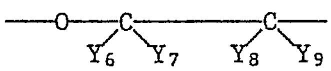 , 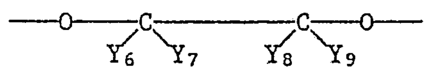 ,

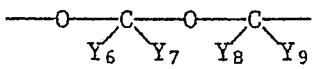 oder 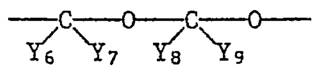

in denen die Substituenten $Y_6$ bis $Y_9$ Wasserstoff, Fluor oder Chlor darstellen, aber mindestens einer dieser Substituenten eine von Wasserstoff verschiedene Bedeutung hat.

Bevorzugte Verbindung der Formel I″ sind diejenigen der folgenden Formeln

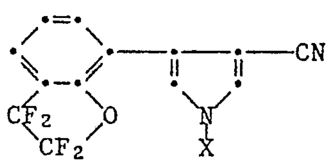

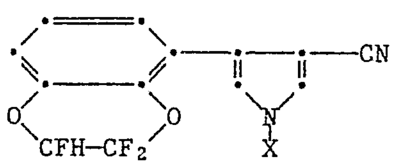 , 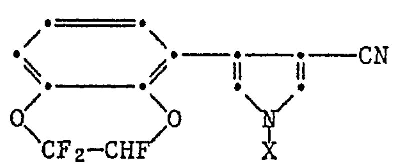

Insbesondere wegen ihrer hervorragenden fungiziden Eigenschaften sind folgende Einzelsubstanzen bevorzugt:
3-(2,3-Bis-trifluormethoxy-phenyl)-4-cyanopyrrol
3-(3-Trifluormethoxy-phenyl)-4-cyanopyrrol
3-(2-Difluormethoxy-phenyl)-4-cyanopyrrol
3-(3-Pentafluorethoxy-phenyl)-4-cyanopyrrol
3-(2,2,3-Trifluor-3-chlor-1,4-benzodioxen-5-yl)-4-cyanopyrrol
3-(2,2-Difluor-1,4-benzodioxen-5-yl)-4-cyanopyrrol
3-(2,2,4,4-Tetrafluor-1,3-benzodioxan-8-yl)-4-cyanopyrrol
3-(1,1,3,3-Tetrafluor-phthalan-4-yl)-4-cyanopyrrol
3-(2,2,3,3-Tetrafluor-cumaran-4-yl)-4-cyanopyrrol
1-Acetyl-3-(2-Difluormethoxy-phenyl)-4-cyanopyrrol.

Bei der erfindungsgemässen Herstellung der Verbindungen der Formel I verfährt man so, dass man

a) zunächst eine Verbindung der Formel Ia durch cyclisierende Michael-Addition eines geeigneten entsprechenden Styrolderivates der Formel II, worin $Y_1$, $Y_2$, $Y_3$, $Y_4$ und $Y_5$ die bei der Definition der Verbindungen der Formel I angegebene Bedeutungen haben, mit p-Toluolsulfonylmethylisocyanid unter Abspaltung von p-Toluolsulfinsäure bzw. ihres Salzes in einem organischen Lösungsmittel in Gegenwart einer Base bei -30 bis +120°C, herstellt,

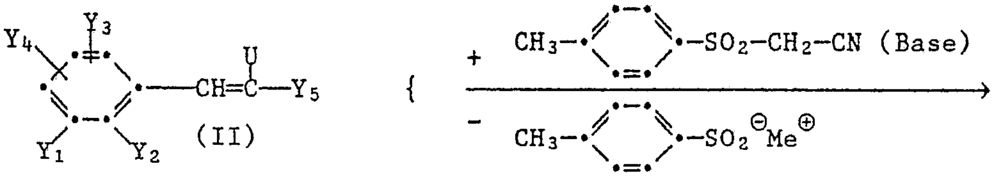


(II)

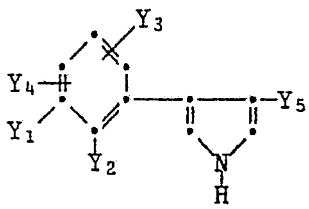

(Ia)

wobei $Me^{\oplus}$ ein alkali- oder Erdalkaliion bedeutet und U für H, $CO_2H$ oder $CO_2R_{20}$ steht, wobei $R_{20}$ $C_1$-$C_6$-Alkyl bedeutet und, falls gewünscht,

b) anschliessend die Verbindung Ia mit Verbindungen der Formel III in Gegenwart eines säurebindenden Mittels und gegebenenfalls eines Katalysators in einem organischen Lösungsmittel

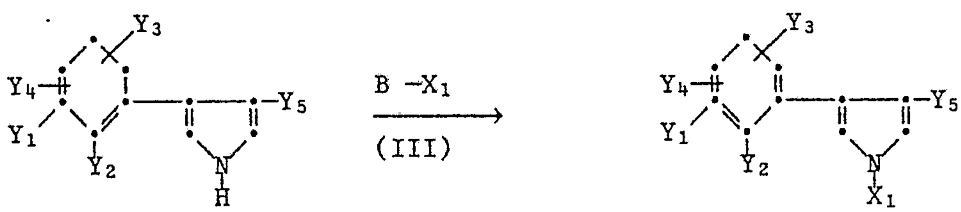


(Ia) (Ib) ,

wobei $X_1$ die unter Formel I, für X, unter B) -G) angegebenen Bedeutungen hat und B für Halogen, OH oder den Rest $OX_1$ steht, umsetzt und in eine Verbindung der Formel Ib überführt.

Reaktionsschritt (a)

Die p-Tolylsulfonyl-Gruppe steht hier stellvertretend für eine ganze Reihe von Gruppen, die in der Lage sind, die Methylengruppe im Methylisocyanid-Rest für eine Reaktion im Sinne einer Michael-Addition zu aktivieren. Weitere bevorzugte Beispiele für derartig aktivierende Gruppen sind Benzolsulfonyl-, p-Chlorbenzolsulfonyl, Niederalkylsulfonyl wie Mesyl.

Die Cycloaddition wird vorteilhafterweise in Gegenwart einer Base durchgeführt. Als Basen eignen sich Alkalimetallhydride wie Natriumhydrid, Alkalimetallhydroxide wie Natriumhydroxid, oder Alkali- bzw. Erdalkalicarbonate, wie $Na_2CO_3$, $K_2CO_3$ oder Alkalialkoholate, wie $(CH_3)_3CO^{\ominus}K^{\oplus}$ und andere und schliesslich metallisches Natrium. Die Base wird vorteilhafterweise in mindestens äquimolarer Menge, bezogen auf die Ausgangssstoffe, eingesetzt.

Für die Cycloaddition ist es zweckmässig, die Reaktion in einem reaktionsinternen Lösungsmittel durchzuführen. Dafür kommen beispielsweise folgende, bevorzugt wasserfreie Lösungsmittel in Frage: Aromatische und aliphatische Kohlenwasserstoffe wie Benzol, Toluol, Xylole, Petrolether, Ligroin, Cyclohexan; Ether und etherartige Verbindungen wie Dialkylether (Diethylether, Diisopropylether, t-Butylmethylether usw.), Dimethoxyethan. Dioxan, Tetrahydrofuran, Anisol; Sulfone wie Dimethylsulfoxid; Dimethylformamid und gemische solcher Lösungsmittel untereinander.

Die Cycloaddition wird im allgemeinen bei Temperaturen von -30° bis +120°C, bevorzugt bei -30° bis +50°C bzw. am Siedepunkt des Lösungsmittels oder Lösungsmittelgemisches, durchgeführt.

Bei der Wahl geeigneter Base lässt sich die Cycloaddition vorteilhafterweise auch in wässrigem Medium ausführen. Als Basen eignen sich in diesen Fällen wasserlösliche anorganische und organische Basen, insbesondere Alkalihydroxide wie LiOH, NaOH oder KOH und Ammoniumbasen, z.B. Tetraalkylammoniumhydroxide wie $(CH_3)_4NOH$. Die Base wird in min destens äquimolarer Menge, bezogen auf die Ausgangsstoffe, eingesetzt. Bei der Verwendung wässriger Basen führt man die Reaktion vorteilhafterweise in einem heterogenen Zweiphasensystem durch.

Für die organische, mit Wasser nicht mischbare Phase kommen z.B. folgende Lösungsmittel in Frage:

Aliphatische und aromatische Kohlenwasserstoffe, wie Pentan, Hexan, Cyclohexen, Petrolether, Ligroin, Benzol, Toluol, Xylole usw., halogenierte Kohlenwasserstoff, wie Dichlormethan, Chloroform, Tetrachlorkohlenstoff, Ethylendichlorid, 1,2-Dichlorethan, Tetrachlorethylen usw., oder aliphatische Ether, wie Diethylether, Diisopropylether, t-Butylmethylether usw.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid-, jodid- usw. Als Phasentransfer-Katalysatoren kommen auch Phosphonium-Salze in Betracht.

Die Phasentransfer-katalysierte Cycloaddition kann bei Temperaturen von 0° bis 80°C, vorzugsweise 10° bis 50°C bzw. beim Siedepunkt des Lösungsmittelgemisches, durchgeführt werden.

Die Cycloaddition kann in den beschriebenen Ausführungsarten bei Normaldruck erfolgen; die Reaktionsdauer beträgt im allgemeinen 1 bis 16 Stunden, bei Phasentransferkatalyse 0,5 bis 10 Stunden.

Reaktionsschritt b)

Die Acylierung der Verbindung Ia wird unter üblichen, dem Fachmann bekannten Bedingungen durchgeführt.

Geeignete Lösungs- oder Verdünnungsmittel sind beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethyl ether, Diisopropylether, tert.-Butylmethylether usw.), Dioxan, Tetrahydrofuran; Nitrile wie Acetonitril, Propionitril; Ketone wie Aceton, Diethylketon, Methylethylketon; und Gemische solcher Lösungsmittel untereinander. Bevorzugt sind Tetrahydrofuran oder Dioxan.

Als säurebindende Mittel eignen sich anorganische Basen, z.B. Oxide, Hydroxide, Carbonate oder Hydrogencarbonate von Alkali- oder Erdalkalimetallen sowie Alkalihydride oder Alkyliacetate, ferner organische Basen, z.B. tertiäre Amine wie Trialkylamine (Trimethylamin, Triethylamin usw.), Pyridin oder Pyridinbasen (4-Dimethylaminopyridin, 4-Pyrrolidylaminopyridin). Bevorzugt sind Trialkylamine wie Trimethylamin oder Triethylamin.

Die Reaktionstemperatur ist je nach den Reaktionsbedingungen variabel. Sie liegt im allgemeinen zwischen -25°C und 100°, vorzugsweise zwischen -10° und 75°C.

Styrolderivate der Formel II, bei welchen Y ≠ H ist, lassen sich durch Knövenagel-Kondensation von Cyanessigsäure oder Cyanessigester mit den entsprechenden Aldehyden herstellen. Solche Umsetzungen sind dem Fachmann bekannt und können analog den Beispielen in Org. React. 15, 204-599 (1967) durchgeführt werden.

Bei der Herstellung der Styrolderivate der Formel II als Ausgangsverbindung für die Verbindungen der Formel Ia geht man von einem entsprechend substituierten Anilin der Formel IV aus, das in der üblichen, dem Fachmann bekannten Weise zum Diazoniumsalz der Formel V umgesetzt wird:

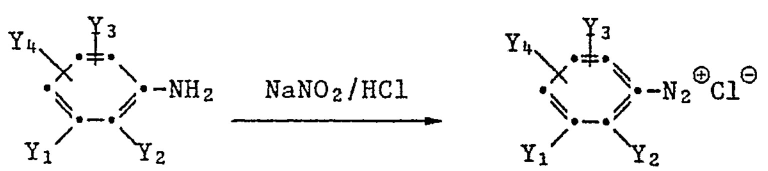


(IV) (V)

Dann lässt man das Diazoniumsalz der Formel V mit dem Alkylen-Derivat der Formel VI in Gegenwart von Cu(I)-Chlorid in einem wässrigen Reaktionsmedium mit einem Dialkylketon als Lösungsvermittler zu dem Anlagerungsprodukt der Formel VII reagieren:

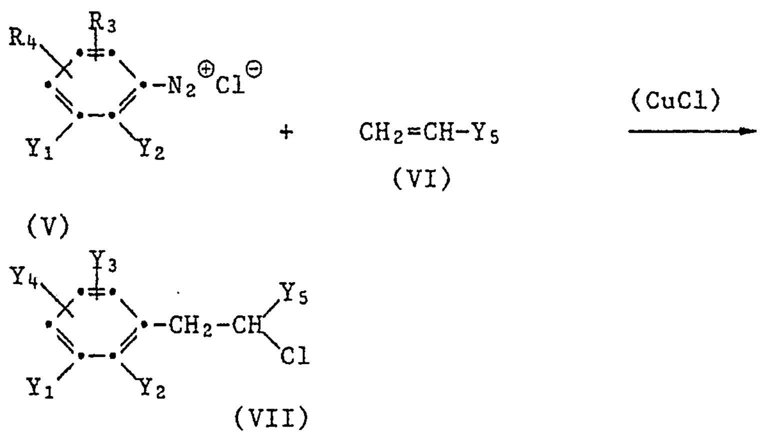


(V) + $CH_2=CH-Y_5$ (VI)

(VII)

Anschliessend wird durch Umsetzung der Verbindung der Formel VII mit einem säurebindenden Mittel in einem inerten organischen Lösungsmittel HCl abgespalten und dabei das entsprechend substituierte Styrol-Derivat (Formel II) erhalten, wobei das Produkt ein cis/trans-Isomerengemisch darstellt, das chromatographisch in üblicher Weise getrennt werden kann:

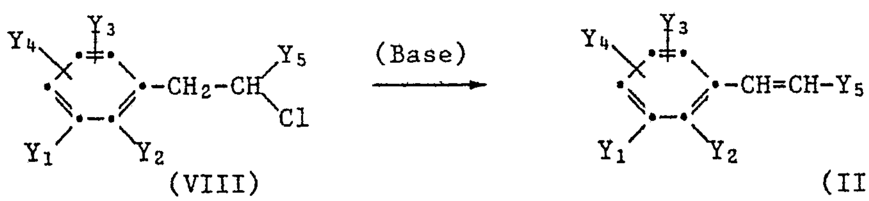


(VIII) (II)

Die durchgeführte Umsetzung des Diazoniumsalzes (Formel V) mit dem Alkylen-Derivat (Formel VI) stellt eine Abwandlung der normalen "Sandmeyer"-Methode durch Awendung der Bedingungen der "Meerwein"-Reaktion von aromatischen Diazonium-Verbindungen auf α-, β-ungesättigte Carbonyl-Verbindungen dar, wodurch der Ersatz der Diazoniumgruppe durch Halogen zu Gunsten der Anlagerungsreaktion zurückgedrängt wird (vgl. E. Müller, Angew. Chemie 61, 178-183, 1949).

In der praktischen Durchführung der Umsetzung werden die Reaktanten Diazoniumsalz und das Alkylen-Derivat im Verhältnis 1:1 bis 1:8, vorzugsweise 1:2, eingesetzt. Die Reaktionstemperaturen betragen 20° bis 50°C, bevorzugt 25°-35°C. Die Reaktionsdauer beträgt 0,5 bis 10 Stunden, vorzugsweise 1 bis 3 Stunden. Als Lösungsvermittler im wässrigen Reaktionsmedium wird bevorzugt Ethylmethylketon eingesetzt.

Bei der Abspaltung von HCl aus der Verbindung der Formel VIII werden als inerte Lösungsmittel beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander verwendet. Als säurebindende Mittel dienen schwach nucleophile organische Basen, vorzugsweise Trialkylamine. Die Abspaltungsreaktion wird bei Temperaturen, die von Raumtemperatur bis zur Rückflusstemperatur des verwendeten Lösungsmittels reichen, bevorzugt von 30° bis 60°C, durchgeführt. Die Reaktionsdauer beträgt 1 bis 24 Stunden, vorzugsweise 3 bis 12 Stunden.

Die Verbindungen der Formel II sind wertvolle Zwischenprodukte zur Herstellung von Fungiziden und stellen als neue Verbindungen einen Teil der Erfindung dar.

3-Phenyl-4-cyanopyrrolderivate sind bereits z. Teil als Fungizide bekannt. Solche Verbindungen werden z.B. in Tetrahedron Letters 52, 5337-5340 (1972) in der Deutschen Offenlegungsschrift DE-OS 29 27 480 und in der EP-A 96142 beschrieben. Die Wirksamkeit der bekannten Derivate hat sich jedoch nicht immer im gewünschten Masse als voll befriedigend erwiesen.

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Mirkoorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an

Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so. z.B. gegen Cercospora, Pyricularia und vor allem gegen Botrytis. Botrytis spp. (B. Cinerea, b. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Dabei weist insbesondere die Verbindung Nr. 1.1 aus Tabelle 1 ein breites Wirkungsspektrum auf. Sie entfaltet nicht nur gegen Pyricularia, Botrytis und Rhizoctonia eine hervorragende fungizide Wirkung, sondern eignet sich auch zur erfolgreichen Bekämpfung von Erysiphe- und Venturia-Species. Ueberdies wirken die Verbindungen systemisch. Darüber hinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftetende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker-und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Cirtrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Compositen).

Im Vorratschutz werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Enkapsulierung in z.B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zeilen und den gegebenen Verhält nissen angepasst. Günstige Aufwandmengen liegen im allgemeinen bei 0,01 bis höchstens 2 kg Aktivsubstanz pro 100 kg zu schützendes Gut; sie hängen jedoch ganz wesentlich von der Beschaffenheit (Grösse der Oberfläche, Konsistenz, Feuchtigkeitsgehalt) des Gutes und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Agrarprodukte genannt: Getreide (wie Weizen, Gerste, Roggen, Hafen, Rise, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him-und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spiinat, Salat, Spargel, Kohlarten, Zwiebeln, Tomaten, Kartoffeln, Paprika): Lorbeergewächse (wie Avocadom Cinnamonum, Kampfer) oder Mias, Tabak, Nüsse, Kaffee, Zuckerrohr, Tee, Weintrauben, Kastanien, Hopfen, Bananen, Gras und Heu.

Als Naturprodukte tierischer Herkunkft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, as Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahen ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tenside oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospholipide.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Boden applikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäugen, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatiscshe Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl; oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht als nicht-sorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionosgene, kation-und/oder anionaktive Tenside mit guten Emulgier-, Dispergier- und Netzeigenschaften in Betracht. Unter Tensiden sind auch Tensidgemische zu verstehen.

Die in der Formuliertungstechnik gebräuchlichen Tenside sind u.a. in folgenden Publikationen beschrieben:

"Mc Cutcheon's Detergents and Emulsifiers Annual" MC Publishing Corp., Ridgewood New Jersey, 1980

Sisley and Wood, "Encyclopedia of Surface Active Agents", Chemical Publishing Co., Inc. New York, 1980.

Die agrochemische Zubereitungen enthalten in der Regel 0,1 bis 99 %, insbesondere 0,1 bis 95 % Wirkstoff der Formel I, 99,9 bis 1 %, insbesondere 99,9 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Derartige (agro)chemische Mittel sind ein Bestandteil der vorliegenden Erfindung.

Die nachfolgenden Beispiele dienen der Illustration der Erfindung, ohne dieselbe einzuschränken (Prozente und Teile beziehen sich auf Gewicht; Temperaturen sind in Celsiusgraden angegeben).

Herstellungsbeispiele

H 1. Herstellung von α-Cyano-2,3-methylendioxy-zimtsäuremethylester

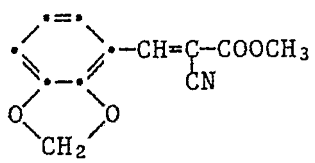

Ein Gemisch von 24,3 g 2,3-Methylendioxy-benzaldehyd 20,8 g Cyanessigsäure methylester, 0,3 g Ammoniumacetat, 3 g Eisessig in 200 ml Toluol wir während 3 Stunden unter Rückfluss erhitzt. Dabei wird das entstehende Reaktionswasser über einen Wasserabscheider abgetrennt. Das Reaktionsgemisch wird anschliessend mit Essigester versetzt, die organische Phase wird je zweimal mit Wasser und Sole gewaschen, über Magnesiumsulfat getrocknet und das Lösungsmittel wird unter vermindertem Druck eingeengt. Das Rohprodukt wird aus Toluol/Hexan umkristallisiert und man erhält die gewünschte Verbindung mit einem Smp. vom 108-110°C.

H 2. Herstellung von 3-(Benzodioxol-4-yl)-4-cyanopyrrol (Verbindung Nr. 1., Tabelle 1)

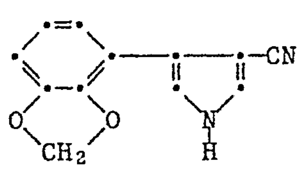

Zu einer Lösung von 34,4 g des obigen α-Cyano-zimtsäureesters in 60 ml Etanol und 200 ml Methylenchlorid gibt man bei einer Temperatur von -5 bis 0°C 10,4 g festes Natriummethylat. Bei derselben Temperatur tropft man innerhalb von 30 Minuten eine Lösung von 30,5 g TOSMIC in 100 ml Methylenchlorid zum Reaktionsgemisch. Nun erwärmt man langsam auf RT und lässt über Nacht nachrühren. Zur Aufarbeitung verdünnt man mit Aether, wäscht die organische Phase mit Wasser und Sole, trocknet sie über $MgSO_4$ und engt sie unter vermindertem Druck ein. Der feste Rückstand wird aus Essigester und Toluol umkristallisiert und man erhält das gewünschte Produkt. Der Schmelzpunkt beträgt 191-194°C.

H 3. Herstellung von 3-Trifluormethoxy-zimtsäurenitril

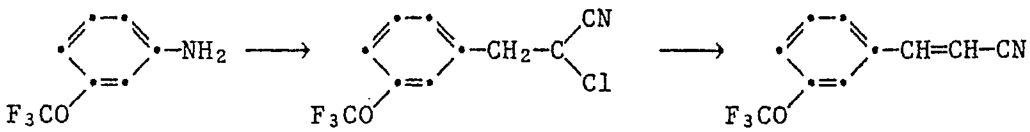

a) Zu einer Lösung von 26 gr 3-Trifluormethoxyanilin in 50 ml Essigsäure werden 36 ml 32%-ige Salzsäue und 4 ml Wasser gegeben. Zum resultierenden Gemisch wird bei 0° eine Lösung von 11.25 g Natriumnitrit in 30 ml Wasser zugetropft und anschliessend noch 1 Stunde bei 0° weitergerührt. Die

entstandene Suspension wird nun bei 26-31° portionenweise zu 20 ml Acrylnitril und 18 ml Aethylmethylketon zufliessen gelassen. Aus einem separaten Tropftrichter wird gleichzeitig eine Lösung von 0,56 g Cu(I)-chlorid in 5,5 ml 32%-iger Salzsäure zugetropft. Nach beendigtem Zutropfen wird noch 30 Min. bei 37° gerührt und dann auf Eis gegossen. Das Gemisch wird zweimal mit Methylenchlorid extrahiert, die organischen Phase zweimal mit verdünnter, eiskalter Natronlauge exttrahiert, über Magnesiumsulfat getrocknet, filtriert und das Filtrat auf ein Volumen von 150 ml eingeengt.

b) Die obige Methylenchlorid-Lösung wird mit 25,5 ml Triethylamin versetzt und 12 Stunden unter Rückfluss erhitzt. Die dunkle Lösung wird nach dem Abkühlen auf Eiswasser gegossen. Die Phasen werden getrennt und die Wasserphase nochmals mit Methylenchlorid extrahiert. Die organischen Phasen werden zweimal mit eiskalter, verdünnter Salzsäure und anschliessend mit halbgesättigter Natriumchlorid-Lösung gewaschen, über Natriumsulfat getrocknet, filtriert und eingeengt. Durch Chromatographie des rohen cis/trans Gemisches (Petroleumbenzin/Ethylacetat 20:1) kann das zur Hauptsache gebildete trans-Isomere des obigen Zimtsäurenitrils in reiner Form erhalten werden. Gelbliche Kristalle Smp. 44-48°.

H 4. Herstellung von 3-(3-Trifluormethoxyphenyl)-4-cyanopyrrol

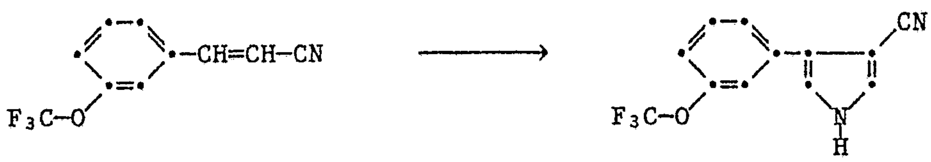

Aus zwei Tropftrichtern werden je eine Lösung von 27.2 g des obigen Zimtsäurenitrils und 31.4 g Tosmic in 200 ml Terahydrofuran und von 20.5 g Kalium-tertiär-butylat in 250 ml Tetrahydrofuran, bei -5° bis +5° in 70 ml Tetrahydrofuran getropft. Anschliessend wird 1 Stunde bei 0° und 2 weitere Stunden bei Raumtemperatur gerührt. Das Reaktionsgemisch wird dann auf Eiswasser gegossen und zweimal mit Aethylacetat extrahiert. Die organischen Extrakte werden viermal mit halbgesättigter Natriumchlorid-Lösung gewaschen, über Natriumsulfat getrocknet, mit Kieselgel, etwas Aktivkohle und Tonsil verrührt, filtriert und eingedampft. Der Rückstand wird aus wenig Methylenchlorid bei -30° kristallisiert und man erhält die Titelverbindung mit einem Smp. 172-177°.

H 5. Herstellung von 1-Acetyl-3-(3-Trifluormethoxyphenyl)-4-cyanopyrrol

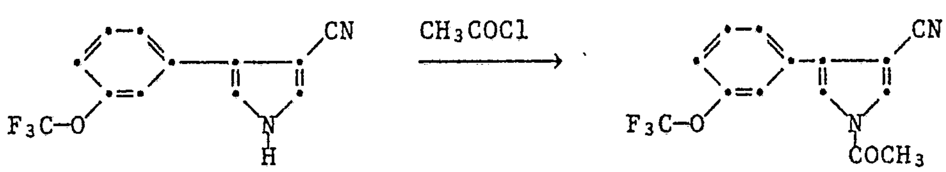


Zu einer Lösung von 3.2 g des obigen Pyrrols in 10 ml Tetrahydrofuran werden 0.2 g 4-Dimethylaminopyridin und 1.9 ml Triethylamin gegeben. Nun wird bei -10° langsam eine Lösung von 1.09 ml Acetylchlorid in 5 ml Tetrahydrofuran zutropfen gelassen. Das Reaktionsgemisch wird 16 Stunden bei auftauendem Eisbad gerührt, filtriert und das Filtrat eingeengt. Der feste Rückstand wird aus Toluol/Petroleumbenzin umkristallisiert. Dabei erhält man die Titelverbindung mit Smp. 110-116°.

Auf analoge Weise werden die in den Tabellen 1-4 angegebenen Verbindungen hergestellt.

Tabelle 1

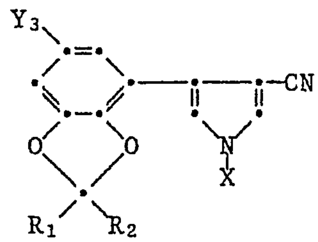

Verbindung

| Nr. | $R_1$ | $R_2$ | $Y_3$ | X | Physik. konst. |
|---|---|---|---|---|---|
| 1.1 | H | H | H | H | Smp.: 191-194°C |
| 1.2 | F | H | H | H | |
| 1.3 | F | Cl | H | H | |
| 1.4 | $CH_3$ | $CH_3$ | H | H | Smp.: 136-138°C |
| 1.5 | $CH_3$ | F | H | H | |
| 1.6 | $CH_3$ | H | H | H | |
| 1.7 | F | F | tert.-$C_4H_9$ | H | Smp.: 158-160°C |
| 1.8 | $CH_3$ | $C_2H_5$ | H | H | |
| 1.9 | $C_3H_7$ | H | H | H | |
| 1.10 | $CF_3$ | $CF_3$ | H | H | |
| 1.11 | $CF_3$ | $CH_3$ | H | H | |
| 1.12 | $CCl_3$ | H | H | H | |
| 1.13 | F | F | H | $SO_2CH_3$ | Smp.: 167-168°C |
| 1.14 | H | H | H | $\underset{\|\|}{C}CH_3$ O | Smp.: 187-189°C |

Tabelle 2

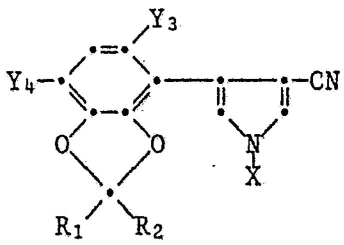

Verbindung

| Nr. | $R_1$ | $R_2$ | $Y_3$ | $Y_4$ | X | Physik. konst. |
|---|---|---|---|---|---|---|
| 2.1 | F | F | Br | Br | H | Smp.:144,5-194°C |

Tabelle 3

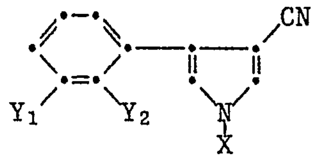

| Nr. | $Y_1$ | $Y_2$ | X | chem.-phys. Daten |
|---|---|---|---|---|
| 3.1 | $OCF_3$ | H | H | Smp. 172-177° |
| 3.2 | $OCF_3$ | Cl | H | Smp. 158-164° |
| 3.3 | $OCF_2H$ | H | H | Smp. 82-84° |
| 3.4 | $OCF_2H$ | Cl | H | Smp. 138-139° |
| 3.5 | $OCF_3$ | $OCF_3$ | H | |
| 3.6 | $OCF_3$ | H | $-COCH_3$ | Smp. 110-116° |
| 3.7 | H | $OCF_3$ | H | Smp. 87-88° |
| 3.8 | H | $OCHF_2$ | H | Smp. 96-97° |
| 3.9 | Cl | $OCF_3$ | H | |
| 3.10 | Cl | $OCHF_2$ | H | |
| 3.11 | $OCF_2CHF_2$ | H | H | |
| 3.12 | $OCF_2CHF_2$ | Cl | H | Smp. 108-109° |
| 3.13 | H | $OCF_2CHF_2$ | H | Smp. 70-72° |
| 3.14 | $OCF_2CHFCl$ | H | H | Smp. 68-70° |
| 3.15 | $OCF_2CHFCl$ | Cl | H | |
| 3.16 | Cl | $OCF_2CHFCl$ | H | |
| 3.17 | $OCF_2CHFCF_3$ | H | H | |
| 3.18 | H | $OCF_2CHFCF_3$ | H | Smp. 80-82° |
| 3.19 | $OCClF_2$ | H | H | |
| 3.20 | $OCBrF_2$ | H | H | |
| 3.21 | H | $OCBrF_2$ | H | |
| 3.22 | $OCHF_2$ | $OCHF_2$ | $-CO-C_6H_5$ | |
| 3.23 | $OCHF_2$ | $OCHF_2$ | $-COCH_3$ | Smp. 68-70° |
| 3.24 | $OCCl_3$ | H | H | |
| 3.25 | H | $OCCl_2CF_3$ | H | |
| 3.26 | H | $OCF_2CFCl_2$ | -CO 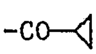 | |
| 3.27 | $OCH_3$ | $OCHF_2$ | H | Smp. 90-93° |

Tabelle 3 (Fortsetzung)

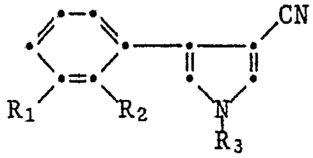

| Nr. | $R_1$ | $R_2$ | $R_3$ | Physik. Daten |
|---|---|---|---|---|
| 3.28 | $OCHF_2$ | $OCHF_2$ | H | $n_D^{22}$ = 1,5300 |
| 3.29 | H | $OCF_2CHFCl$ | H | 65-67°C |
| 3.30 | $OCH_3$ | $OCF_2CHFCl$ | H | 90-92°C |
| 3.31 | $OCH_3$ | $OCF_2CHF_2$ | H | 89-90°C |
| 3.32 | $OCH_3$ | $OCHF_2$ | 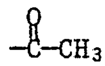 | 116-118° |
| 3.33 | $OCH_3$ | $OCF_2CHFCl$ | 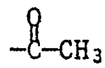 | Harz |
| 3.34 | $OCH_3$ | $OCF_2CHF_2$ | 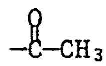 | Harz |
| 3.35 | $OCF_2CHFCl$ | $OCF_2CHFCl$ | H | 109-110° |

Tabelle 4

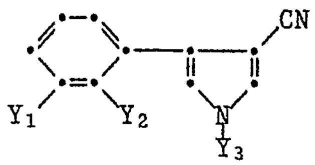

| Nr. | $Y_1$ $Y_2$ | X | chem.-phys. Daten |
|---|---|---|---|
| 4.1 | $-O-CF_2-CF_2-O-$ | H | |
| 4.2 | $-O-CF_2-CF_2-O-$ | $-COCH_3$ | |
| 4.3 | $-O-CF_2-CFCl-O-$ | H | |
| 4.4 | $-O-CFCl-CF_2-O-$ | H | |
| 4.5 | $-O-CF_2-O-CF_2-$ | H | |
| 4.6 | $-CF_2O--CF_2O-$ | H | |
| 4.7 | $-CF_2-O-CF_2-$ | H | |
| 4.8 | $-O-CF_2-CF_2-$ | H | |
| 4.9 | $-CF_2-CF_2-O-$ | H | Smp.: 183-186° |
| 4.10 | $-O-CF_2-CH_2O-$ | H | Smp.: 128-130° |
| 4.11 | $-O-CF_2-CHF-O-$ | H | Smp.: 174-176° |
| 4.12 | $-OCH_2-CF_2-O-$ | H | |
| 4.13 | $-O-CHF-CF_2-O-$ | H | Smp.: 119-122° |
| 4.14 | $-CF_2-O-CFCF_3-O-$ | H | |
| 4.15 | $-O-CCl_2-CCl_2-O-$ | H | |
| 4.16 | $-O-CCl_2-CCl_2-$ | H | |

Formulierungsbeispiele für Wirkstoffe der Formel I (% = Gewichtsprozent)

F 1. Emulsion-Konzentrate

| | a) | b) | c) |
|---|---|---|---|
| Wirkstoff aus Tabellen 1-4 | 25% | 40% | 50% |
| Ca-Dodecylbenzolsulfonat | 5% | 8% | 6% |
| Ricinusöl-polyäthylenglykoläther (36 Mol Aethylenoxid | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

F 2. Lösungen

| | a) | b) | c) | d) |
|---|---|---|---|---|
| Wirkstoff aus Tabellen 1-4 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

F 3. Granulate

| | a) | b) |
|---|---|---|
| Wirkstoff aus Tabellen 1-4 | 5% | 10% |
| Kaolin | 94% | |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

F 4. Stäubemittel

| | a) | b) |
|---|---|---|
| Wirkstoff aus Tabellen 1-4 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

F 5. Spritzpulver

| | a) | b) | c) |
|---|---|---|---|
| Wirkstoff aus Tabellen 1-4 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

Biologische Beispiele:

B1.: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Ureidosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Ureidosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Verbindungen aus den Tabellen 1-4 zeigten gegen Puccinia-Pilze eine gute Wirkung. Unbehandelte aber infizierte Kontrollpflanzen zeigten einen Puccinia-Befall von 100 %. Unter anderen hemmten die Verbindungen Nr. 1.1, 1.7, 2.1, 3.1, 3.3 oder 3.6 den Puccinia-Befall auf 0 bis 5 %.

B 2.: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,006 % Aktivsubstanz besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1-4 behandelt wurden, einen stark reduzierten Cercospora-Befall. So verhinderten die Verbindungen Nr. 1.1, 1.7, 2.1, 3.1, 3.2 und 3.6 in obigen Versuchen das Auftreten von Flecken fast vollständig.

B 3.: Wirkung gegen Erysiphe graminis auf Gerste

Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt. Verbindungen aus den Tabellen 1-4 waren gut wirksam gegen Erysiphe-Befall von Gerste.

B 4.: Residual-protektive Wirkung gegen Venturia inaequalis auf Apfeltrieben.

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24° C aufgestellt. Der Schorbefall wird 1-5 Tage nach der Infektion beurteilt. Wirkstoffe aus den Tabellen 1-4 zeigten gute Wirksamkeit gegen Venturia auf Apfeltrieben. Verbindungen Nr. 1.1, 1.7, 2.1, 3.1, 3.2 und 3.6 hemmten den Krankheitsbefall auf weniger als 10 %. Unbehandelte aber infizierte Triebe zeigten einen 100 %igen Venturia-Befall.

B 5.: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0k02 % Aktivsubstanz) besprüht. Nach 48 Stunden werden die behandelten Pflanzen mit einer Konidiensuspension des Pilzes infiziert. Nach einer inkubation der infizierten Pflanzen während 3 Tagen bei 95-100 % relativer Luftfeuchtigkeit und 21° C erfolgt die Beurteilung des Pilzbefalls. Die Verbindungen aus den Tabellen 1-4 hemmten in vielen Fällen die Pilzinfektion sehr stark. Bei einer Konzentration von 0.02 % erwiesen sich z.B. der Verbindungen Nr. 1.1, 1.7, 2.1, 3.1, 3.2 und 3.6 als voll wirksam (Krankheitsbefall 0 bis 5 %). Befall unbehandelter aber infizierter Bohnenpflanzen betrug 100 %.

B 6.: Wirkung gegen Botrytis cinerea an Apfelfrüchten

Künstlich verletzte Aepfel werden behandelt, indem eine aus Spritzpulver der Wirksubstanz hergestellte Spritzbrühe auf die Verletzungsstellen aufgetropft wird. Die behandelten Früchte werden anschliessend mit

einer Sporensuspension von Botrytis cinerea inokuliert und während einer Woche bei einer hohen Luftfeuchtigkeit und ca. 20°C inkubiert.

Bei der Auswertung werden die angefaulten Verletzungsstellen gezählt und daraus die fungizide Wirkung der Testsubstanz abgeleitet. Verbindungen aus den Tabellen 1-4 waren gut wirksame gegen Botrytis-befall von Apfelfrüchten. Unter anderen hemmten die Verbindungen Nr. 1.1., 1.7, 2.1, 3.1, 3.2 und 3.6 den Pilzbefall im Vergleich zu unbehandelten Kontrollfrüchten (mit 100 % Befall) fast vollständig.

B 7.: Wirkung gegen Alternaria solani auf Tomate

Tomatenpflanzen werden nach dreiwöchiger Anzucht mit einer aus Spritzpulver des Wirkstoffs hergestellten Spritzbrühe (0,06 % Aktivsubstanz) besprüht. Nach 24 Stunden werden die Tomatenpflanzen mit einer Konidiensuspension des Pilzes infiziert. Die Beurteilung der fungiziden Wirkung erfolgt aufgrund des Pilzbefalls nach einer Inkubation der infizierten Pflanzen während 8 Tagen bei hoher Luftfeuchtigkeit und einer Temperatur von 18 - 22°C. Verbindungen aus den Tabellen 1-4 bewirkten eine stark Reduktion des Alternariabefalls; so hemmten die Substanzen Nr. 1.1, 1.7, 2.1, 3.1, 3.2 und 3.6 den Befall vollständig (0-5%).

B 8.: Wirkung gegen Pyricularia oryzae auf Reis

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt. Verbindungen aus den Tabellen 1-4 zeigten eine gute Hemmung des Pyriculariabefalls, so reduzierten z.B. die Verbindungen Nr. 1.1, 1.7, 2.1 den Befall auf weniger als 10 %.

B 9.: Wirkung gegen Fusarium nivale auf Roggen

Auf natürliche Weise mit Fusarium nivale infizierter Roggen der Sorte Tetrahell wird auf einer Mischrolle mit dem zu prüfenden Fungizid gebeizt, wobei eine Konzentration von 60 ppm AS (bezogen auf das Gewicht des Saatgutes) zur Anwendung gelangt. Der infizierte und behandelte Roggen wird im Oktober im Freiland mittels einer Sämaschine auf Parzellen von 3 m Länge und 6 Saatreihen mit dreifacher Wiederholung ausgesät. Bis zur Befallsauswertung wird die Versuchspflanzung unter normalen Feldbedingungen kultiviert (vorzugsweise in einer Region mit geschlossener Schneedecke während der Wintermonate). Zur Ermittlung der Wirkstoffaktivität wird im Frühjahr, unmittelbar nach der Schneeschmelze, der prozentuale Anteil Fusarium-befallener Pflanzen ausgezählt. Verbindungen aus den Tabellen 1-4 zeigten in diesem Versuch gute Wirksamkeit gegen Fusarium auf Roggen, z.B. die Verbindungen Nr. 3.1, 3.2 und 3.6. Unbehandelte jedoch infizierte Kontrollpflanzen wiesen dagegen einen Fusarium-Befall von 100 % auf.

B 10.: Wirkung gegen Helminthosporium gramineum an Gerste

Auf natürliche Weise mit Helminthosporium gramineum infizierte Wintergerste der Sorte "Cl" wird auf einer Mischrolle mit dem zu prüfenden Fungizid gebeizt, wobei eine Konzentration von 60 ppm AS (bezogen auf das Gewicht des Saatgutes) zur Anwendung gelangt. Die infizierte und behandelte Gerste wird im Oktober im Freiland mittels einer Sämaschine auf Parzellen von 2 m Länge und 3 Saatreihen mit dreifacher Wiederholung ausgesät. Bis zur Befallsauswertung wird die Versuchspflanzung unter normalen Feldbedingungen kultiviert. Zur Ermittlung der Wirkstoffaktivität werden zum Zeitpunkt des Aehrenschiebens der prozentuale Anteil Helminthosporium-befallener Halme ausgezählt. Verbindungen aus den Tabellen 1-4 zeigen in diesem Versuch gute Wirksamkeit gegen Helminthosporium. Unbehandelt jedoch infizierte Kontrollpflanzen wiesen dagegen einen Helminthosporium-Befall von 100 % auf.

B 11.: Wirkung gegen Tilletia caries an Weizen

Künstlich mit Brandsporen von Tilletia caries infizierte Winterweizen der Sorte Probus (3 g trockenes Sporenmaterial auf 1 kg Saatgut) wird auf einer Mischrolle mit dem zu prüfenden Fungizid gebeizt, wobei eine Konzentration von 60 ppm AS (bezogen auf das Gewicht des Saatgutes) zur Anwendung gelangt. Der infizierte und behandelte Weizen wird im Oktober im Freiland mittels einer Sämaschine auf Parzellen von 2 m Länge und 3 Saatreihen mit dreifacher Weiderholung ausgesät. Zur Ermittlung der Wirkstoffaktivität werden zum Zeitpunkt der Aehrenreife der prozentuale Anteil Tilletia-befallener Aehren ausgezählt.

Verbindungen aus den Tabellen 1-4 zeigten in diesem Versuch gute Wirksamkeit gegen Tilletia. Unbehandelt jedoch infizierte Kontrollpflanzen wiesen dagegen einen Tilletia-Befall von 100 % auf.

**Patentansprüche**

1. Verbindungen der Formel I

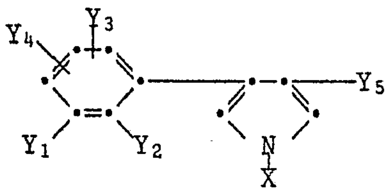

(I),

worin einer der beiden Substituenten $Y_1$ oder $Y_2$ eine $C_1$-$C_3$-Haloalkoxy-Gruppe und der andere Wasserstoff, Halogen, $C_1$-$C_3$-Alkoxy oder eine $C_1$-$C_3$-Haloalkoxy-Gruppe bedeutet, oder worin $Y_1$ und $Y_2$ zusammen mit den beiden Kohlenstoffatomen des Phenylrings einen unsubstituierten oder $C_1$-$C_3$-Alkyl-, $C_1$-$C_3$-Haloalkyl- oder halogensubstituierten 5-Ring-Heterocyclus mit einem oder zwei Sauerstoffatomen oder einen halogensubstituierten 6-Ring-Heterocyclus mit zwei Sauerstoffatomen,
$Y_3$ und $Y_4$ unabhängig voneinander Wasserstoff, Halogen, $C_1$-$C_6$-Alkyl, $C_1$-$C_6$-Alkenyl, $NO_2$, $NH_2$, $CF_3$, CN oder $C_1$-$C_4$-Aliphatoxy bedeuten, und
$Y_5$ für Cl, $CF_3$ oder CN steht, während
X eine der folgenden Bedeutungen hat:

A) Wasserstoff,

B) einen Sulfonsäurerest $SO_2$-R, wobei R unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy oder $C_3$-$C_6$-Cycloalkyl, unsubstituiertes oder durch Halogen, $NO_2$, $C_1$-$C_4$-Alkyl oder $C_1$-$C_4$-Alkoxy substituiertes Phenyl darstellt;

C) Wasserstoff oder den Acylrest einer Carbonsäure;

D) einen Rest S-$R_6$, worin $R_6$ $C_1$-$C_3$-Haloalkyl bedeutet;

E) einen Rest

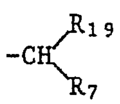

wobei $R_7$ für Wasserstoff oder $C_1$-$C_8$-Haloalkyl $R_{19}$ für Hydroxy, Halogen oder OC(O)$R_8$ stehen, worin $R_8$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

F) einen Rest $CH_2$-Z, wobei Z für eine der Gruppen

a) 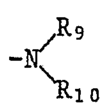 oder b) 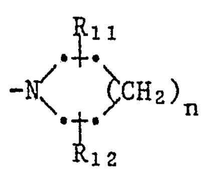 oder c) 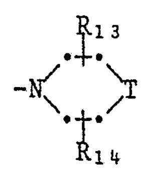

steht, worin $R_9$ und $R_{10}$ unabhängig voneinander Wasserstoff, unsubstituiertes oder durch Cyano, $C_1$-$C_6$-Alkoxycarbonyl substituiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_9$ oder $R_{10}$ Wasserstoff sein kann; $R_{11}$ und $R_{12}$ unabhängig voneinander für Wasserstoff, $C_1$-$C_6$-Alkyl, $C_1$-$C_6$-Alkoxycarbonyl darstellen oder beide zusammen einen ankondensierten aromatischen Ring bilden; $R_{13}$ und $R_{14}$ unabhängig voneinander für Wasserstoff, $C_1$-$C_6$-Alkyl oder $C_1$-$C_6$-Alkoxycarbonyl stehen; und T Sauerstoff, Schwefel, >C=O oder >N-$R_{15}$ bedeutet;
worin $R_{15}$ Wasserstoff, $C_1$-$C_6$-Alkyl, Formyl; $C_1$-$C_6$-Alkanoyl oder $C_1$-$C_6$-Alkoxycarbonyl darstellt; und n für eine der Zahlen 0 oder 1 steht;

G) einen Silylrest -Si($R_{16}$)($R_{17}$)($R_{18}$), wobei $R_{16}$, $R_{17}$ und $R_{18}$ unabhängig voneinander für $C_1$-$C_4$-Alkyl, Benzyl oder Phenyl stehen; wobei X stets einen Sulfonsäurerest oder einen Silylrest bedeutet, wenn gleichzeitig $Y_1 = Y_2 = F$ und $Y_3 = Y_4 = H$ und $Y_5$ = Cyano oder gleichzeitig $Y_1$ und $Y_2$ zusammen -O-$CF_2$-O- und $Y_3 = Y_4 = H$ und $Y_5$ = Cyano sind.

2. Verbindungen der Formel I′

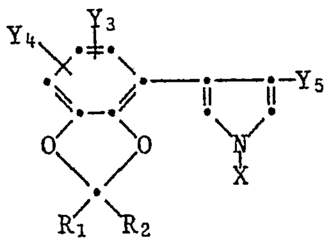 (I'),

gemäss Anspruch 1, worin
$R_1$ und $R_2$ unabhängig voneinander Wasserstoff, Fluor, Chlor, $C_1$-$C_3$-Alkyl, $C_1$-$C_3$-Haloalkyl bedeuten, während X eine der folgenden Bedeutungen hat:

A) Wasserstoff,

B) einen Sulfonsäurerest $SO_2$-R, wobei R unsubstituiertes $C_1$-$C_6$-Alkyl oder durch Halogen oder $C_1$-$C_3$-Alkoxy substituiertes $C_1$-$C_6$-Alkyl; $C_3$-$C_6$-Alkenyl, $C_3$-$C_6$-Alkinyl, unsubstituiertes $C_1$-$C_6$-Alkoxy oder durch Halogen oder $C_1$-$C_3$-Alkoxy substituiertes $C_1$-$C_6$-Alkoxy; $C_3$-$C_6$-Alkenyloxy oder $C_3$-$C_6$-Cycloalkyl darstellt;

C) einen Carbosäurerest CO-R, wobei R die unter B) genannte Bedeutung hat, ein Tetrahydrofuryl(2)-Rest ist oder einen unsubstituierten oder durch Halogen, $NO_2$, $C_1$-$C_4$-Alkyl oder $C_1$-$C_4$-Alkoxy substituierten Phenyl-oder Benzylrest darstellt.

D) einen Rest S-$R_6$, worin $R_6$ $C_1$-$C_3$-Haloalkyl bedeutet;

E) einen Rest

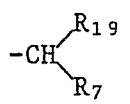

wobei $R_7$ für Wasserstoff oder $C_1$-$C_8$-Haloalkyl $R_{19}$ für Hydroxy, Halogen oder OC(O)$R_8$ stehen, worin $R_8$ $C_1$-$C_8$-Alkyl, $C_1$-$C_8$-Haloalkyl, $C_2$-$C_6$-Alkenyl, 2-Tetrahydrofuryl, 2-Tetrahydropyranyl oder $C_1$-$C_6$-Alkoxycarbonyl darstellen;

F) einen Rest $CH_2$-Z, wobei Z für eine der Gruppen

a) 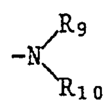 oder b) 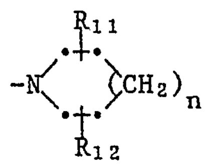 oder c) 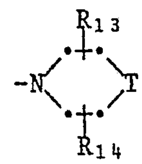

steht, worin $R_9$ und $R_{10}$ unabhängig voneinander Wasserstoff, unsubstituiertes oder durch Cyano, $C_1$-$C_6$-Alkoxycarbonyl substitutiertes $C_1$-$C_6$-Alkyl; $C_3$-$C_6$-Alkenyl, $C_3$-$C_6$-Alkinyl, $C_3$-$C_7$-Cycloalkyl, unsubstituiertes oder durch Halogen, $C_1$-$C_6$-Alkyl, $C_1$-$C_6$-Haloalkyl und/oder $C_1$-$C_6$-Alkoxy substituiertes Phenyl bedeuten, mit der Massgabe, dass nur $R_9$ oder $R_{10}$ Wasserstoff sein kann; $R_{11}$ und $R_{12}$ unabhängig voneinander für Wasserstoff, $C_1$-$C_6$-Alkyl, $C_1$-$C_6$-Alkoxycarbonyl darstellen oder beide zusammen einen ankondensierten aromatischen Ring bilden; $R_{13}$ und $R_{14}$ unabhängig voneinander für Wasserstoff, $C_1$-$C_6$-Alkyl oder $C_1$-$C_6$-Alkoxycarbonyl stehen; und T Sauerstoff, Schwefel, $>C=O$ oder $>N$-$R_{15}$ bedeutet;
worin $R_{15}$ Wasserstoff, $C_1$-$C_6$-Alkyl, Formyl; $C_1$-$C_6$-Alkanoyl oder $C_1$-$C_6$-Alkoxycarbonyl darstellt; und n für eine der Zahlen 0 oder 1 steht;

G) einen Silylrest -Si($R_{16}$)($R_{17}$)($R_{18}$), wobei $R_{16}$, $R_{17}$ und $R_{18}$ unabhängig voneinander für $C_1$-$C_4$-Alkyl, Benzyl oder Phenyl stehen; wobei X stets einen Sulfonsäurerest oder einen Silylrest bedeutet, wenn gleichzeitig $R_1 = R_2 = F$ und $Y_3 = Y_4 = H$ und $Y_5$ = Cyano sind.

3. Verbindungen der Formel I′ gemäss Anspruch 2, dadurch gekennzeichnet, dass
$R_1$ Wasserstoff, Fluor, Chlor, $C_1$-$C_3$-Alkyl, $C_1$-$C_3$-Haloalkyl,
$R_2$ Wasserstoff, Chlor, $C_1$-$C_3$-Alkyl, $C_1$-$C_3$-Haloalkyl, und
$Y_3$ und $Y_4$ unabhängig voneinander Wasserstoff, Halogen, $C_1$-$C_4$-Alkyl, $NO_2$, $NH_2$, $CF_3$, oder $C_1$-$C_4$-Aliphatoxy bedeuten,
$Y_5$ für Cl, $CF_3$ oder CN steht, während

X eine der folgenden Bedeutungen hat:

A) Wasserstoff;

B) einen Sulfonsäurerest $SO_2$-R, wobei R $C_1$-$C_4$-Alkyl, Phenyl, Chlorphenyl oder Tolyl ist;

C) einen Carbosäurerest CO-R, wobei R $C_1$-$C_6$-Alkyl, $C_1$-$C_4$-Haloalkyl oder durch Halogen oder Methyl substituiertes Phenyl oder Benzyl oder unsubstituiertes Phenyl oder Benzyl bedeutet.

D) einen Rest S-$R_6$, worin $R_6$ $C_1$-$C_3$-Haloalkyl ist;

E) einen Rest

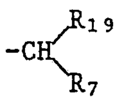

worin $R_7$ für Wasserstoff oder $C_1$-$C_8$-Haloalkyl

$R_{19}$ für Hydroxy, Halogen oder O-CO-$R_8$ bedeuten, mit $R_8$ $C_1$-$C_6$-Alkyl oder $C_1$-$C_4$-Haloalkyl;

F) einen Rest $CH_2$-Z, worin Z -$NHR_{10}$ oder -N($R_9$),($R_{10}$) darstellt, mit $R_9$ und $R_{10}$ = $C_1$-$C_6$-Alkyl;

G) einen Silylrest -Si($R_{16}$)($R_{17}$)($R_{18}$), worin $R_{16}$, und $R_{17}$ unabhängig voneinander für $C_1$-$C_4$-Alkyl stehen und $R_{18}$ $C_1$-$C_4$-Alkyl, Benzyl oder Phenyl bedeutet.

4. Verbindungen der Formel I′ gemäss Anspruch 3, dadurch gekennzeichnet, dass
$R_1$Fluor, Chlor, Methyl, Ethyl, $CF_3$,
$R_2$ Wasserstoff, Chlor, $C_1$-$C_3$-Alkyl, $CF_3$ und
$Y_3$ und $Y_4$ unabhängig voneinander Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeuten.

5. Verbindungen der Formel I′ gemäss Anspruch 4, dadurch gekennzeichnet, dass X Wasserstoff bedeutet.

6. Verbindungen der Formel I′ gemäss Anspruch 5, dadurch gekennzeichnet, dass $Y_3$ Wasserstoff ist und $Y_4$ in meta-Stellung zum Pyrrolsubstituenten steht und Wasserstoff Halogen, $NO_2$, $NH_2$, $CH_3$, iso$C_3H_7$ oder tert.$C_4H_9$ bedeutet.

7. Verbindungen der Formel I′ gemäss Anspruch 6, dadurch gekennzeichnet, dass $R_1$ Fluor, Chlor, $CF_3$ oder Methyl und $R_2$ Wasserstoff, Chlor, $CF_3$, Methyl oder Ethyl bedeuten, $Y_5$ für CN oder $CF_3$ steht.

8. Verbindungen der Formel I′ gemäss Anspruch 7, worin $Y_5$ Cyano bedeutet.

9. Verbindungen der Formel I′ gemäss Anspruch 2, dadurch gekennzeichnet, dass $R_1 = R_2$ = Fluor ist, $Y_3$ Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeutet, $Y_4$ Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeutet und $Y_5$ Cyano darstellt, während X die im Anspruch 3 angegebene bedeutung hat.

10. Verbindungen der Formel I′ gemäss Anspruch 9, dadurch gekennzeichnet, dass $Y_3$ Wasserstoff ist und $Y_4$ in meta-Stellung zum Pyrrolsubstituenten steht und Halogen, $NO_2$, $NH_2$, $CH_3$, iso$C_3H_7$ oder tert.$C_4H_9$ bedeutet, während X Wasserstoff ist und $R_1 = R_2$ = Fluor und $Y_5$ Cyano darstellen.

11. Verbindungen der Formel I′ gemäss Anspruch 2, dadurch gekennzeichnet, dass $R_1$ und $R_2$ unabhängig voneinander Wasserstoff, Fluor, Chlor, $C_1$-$C_3$-Alkyl oder $C_1$-$C_3$-Haloalkyl darstellen, $Y_3$ und $Y_4$ unabhängig voneinander Wasserstoff, Halogen, $C_1$-$C_4$-Alkyl, $NO_2$, $NH_2$, $C_1$-$C_4$-Alkoxy oder Alkenyloxy oder Alkinyloxy mit maximal 3 C-Atomen bedeuten, $Y_5$ $CF_3$ ist und X die im Anspruch 3 angegebene Bedeutung hat.

12. Verbindungen der Formel I′ gemäss Anspruch 11, worin $Y_3$ Wasserstoff ist und $Y_4$ in meta-Stellung zum Pyrrolsubstituenten steht und Wasserstoff, Halogen, $NO_2$, $NH_2$, $CH_3$, iso$C_3H_7$ oder tert.$C_4H_9$ bedeutet, X Wasserstoff darstellt.

13. Verbindungen der Formel I′ gemäss Anspruch 2, worin $R_1$ und $R_2$ unabhängig voneinander Wasserstoff, Fluor, Chlor, $C_1$-$C_3$-Alkyl oder Haloalkyl darstellen, $Y_3$ und $Y_4$ Wasserstoff, Halogen, $C_1$-$C_4$-Alkyl, $NO_2$, $NH_2$, $C_1$-$C_4$-Alkoxy, Alkenyloxy oder Alkinyloxy, jeweils mit maximal 3 C-Atomen bedeuten und $Y_5$ Chlor darstellt, während X Wasserstoff ist.

14. Verbindungen der Formel I′ gemäss Anspruch 13, worin $Y_3$ Wasserstoff ist und $Y_4$ in meta-Stellung zum Pyrrolsubstituenten steht und Wasserstoff, Halogen, $NO_2$, $NH_2$, $CH_3$, iso$C_3H_7$ oder tert.$C_4H_9$ bedeutet, X Wasserstoff darstellt.

15. Verbindungen der Formel I″ gemäss Anspruch 1,

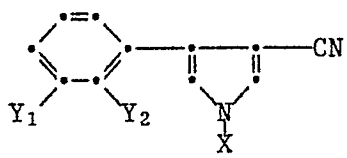

(I")

worin einer der beiden Substituenten $Y_1$ oder $Y_2$ eine $C_1$-$C_3$-Haloalkoxy-Gruppe und der andere Wasserstoff, Halogen, $C_1$-$C_3$-Alkoxy oder eine $C_1$-$C_3$-Haloalkoxy-Gruppe bedeutet, oder worin $Y_1$ und $Y_2$ zusammen mit den beiden Kohlenstoffatomen des Phenylrings einen halogensubstituierten 5-Ring-Heterocyclus mit einem Sauerstoffatom oder einen halogensubstituierten 6-Ring-Heterocyclus mit 2 Sauerstoffatomen bedeuten.

16. Verbindungen gemäss Anspruch 15, worin X für Wasserstoff oder den Acylrest einer $C_1$-$C_8$ aliphatischen oder $C_1$-$C_8$ araliphatischen Carbonsäure oder für einen gegebenenfalls substituierten Benzoylrest steht.

17. Verbindungen gemäss Anspruch 16, worin X einen Benzoylrest darstellt, der durch Halogen, $C_1$-$C_4$-Alkyl, $C_1$-$C_4$-Alkoxy oder $-CF_3$ substituiert sein kann.

18. Verbindungen gemäss Anspruch 15, worin die Zahl der Halogenatome der $C_1$-$C_3$-Halo-alkoxygruppen und der heterocyclischen Ringe 1 bis n beträgt, wobei n die Zahl der maximal möglichen Substitutionen in dem betreffenden Rest bedeutet.

19. Verbindungen gemäss Anspruch 15, worin Fluor und Chlor die Halogensubstituenten in den angegebenen Resten sind.

20. Verbindungen gemäss Anspruch 19, worin Fluor der alleinige Halogensubstituent in den angegebenen Resten ist.

21. Verbindungen gemäss Anspruch 15, worin sowohl $Y_1$ wie $Y_2$ ein4 $C_1$-$C_3$-Haloalkoxy-Gruppe bedeutet.

22. Verbindungen gemäss Anspruch 15, worin entweder $Y_1$ oder $Y_2$ eine $C_1$-$C_3$-Haloalkoxy-Gruppe bedeutet.

23. Verbindungen gemäss einem der Ansprüche 15, 21 oder 22, worin die $C_1$-$C_3$-Haloalkoxy-Gruppe eine der folgenden Bedeutungen hat:

| | | |
|---|---|---|
| A) $-OCHF_2$ | H) $-OCH_2-CF_3$ | O) $-OCF_3$ |
| B) $-OCF_2-CHF_2$ | I) $-OCH_2-CH_2Cl$ | P) $-OCH_2Cl$ |
| C) $-OCF_2-CFCl_2$ | J) $-OCH_2-CH_2F$ | Q) $-OCHFCl$ |
| D) $-OCF_2-CHCl_2$ | K) $-OCH_2-CCl_3$ | R) $-OCH_2F$ |
| E) $-OCF_2-CHFCl$ | L) $-OCF_2-CHF-CF_3$ | S) $-OCClF_2$ |
| F) $-OCF_2-CCl_3$ | M) $-OCCl_3$ | T) $-OCF_2CF_2Cl$ |
| G) $-OCBrF_2$ | N) $-OC_2F_5$ | |

24. Verbindungen gemäss Anspruch 23, worin die Haloalkoxygruppe die Bedeutungen A), B), C), E) oder O) hat.

25. Verbindungen der Formel I" gemäss Anspruch 15, worin $Y_1$ und $Y_2$ zusammen eines der Ringglieder

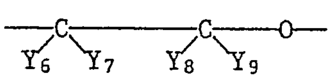 , 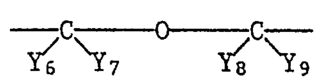 ,

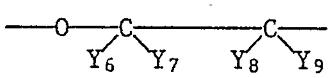 , 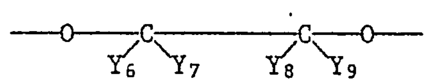 ,

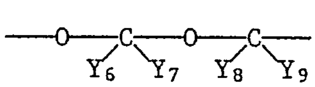 oder 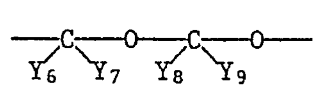

bedeuten, in denen die Substituenten $Y_6$ bis $Y_9$ Wasserstoff, Fluor oder Chlor darstellen, aber mindestens

einer dieser Substituenten eine von Wasserstoff verschiedene Bedeutung hat.

26. Verbindungen gemäss Anspruch 25, der Formel

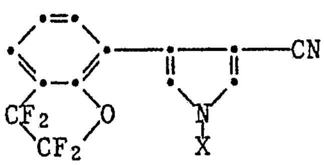

27. Verbindungen gemäss Anspruch 25, der Formeln

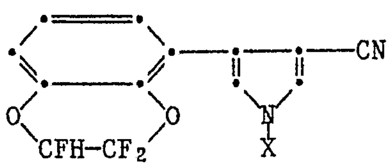

,

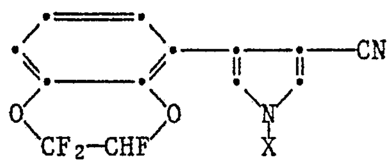

28. Verfahren zur Herstellung der Verbindungen der Formel I in Anspruch 1, dadurch gekennzeichnet, dass man

a) zunächst eine Verbindung der Formel Ia durch cyclisierende Michael-Addition eines geeigneten entsprechenden Styrolderivates der Formel II, worin $Y_1$, $Y_2$, $Y_3$, $Y_4$ und $Y_5$ die bei der Definition der Verbindungen der Formel I angegebene Bedeutungen haben, mit p-Toluolsulfonylmethylisocyanid unter Abspaltung von p-Toluolsulfinsäure bzw. ihres Salzes in einem organischen Lösungsmittel in Gegenwart eines Base bei -30 bis +120°C, herstellt,

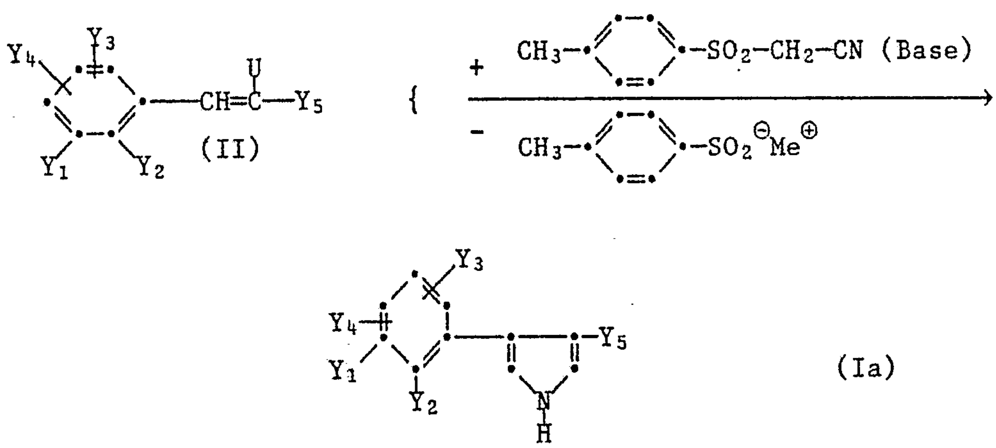


(II)

(Ia)

wobei $Me^{\oplus}$ ein Alkali- oder Erdalkaliion bedeutet und U für H, $CO_2H$ oder $CO_2R_{20}$ steht, wobei $R_{20}$ $C_1$-$C_6$-Alkyl bedeutet und, falls gewünscht,

b) anschliessend die Verbindung Ia mit Verbindungen der Formel III in Gegenwart eines säurebindenden Mittels und gegebenenfalls eines Katalysators in einem organischen Lösungsmittel

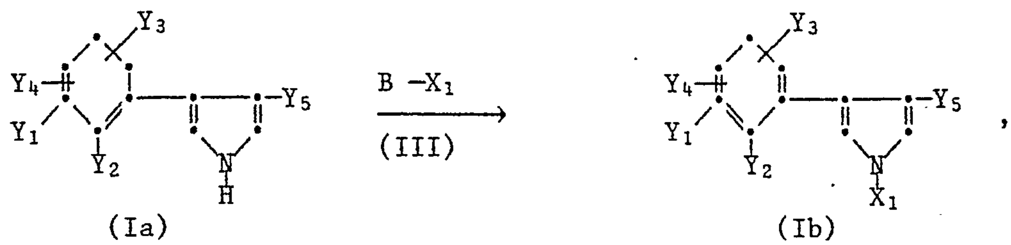

wobei $X_1$ die unter Formel I, für X, unter B) -G) angegebenen Bedeutungen hat und B für Halogen, OH oder den Rest $OX_1$ steht, umsetzt und in eine Verbindung der Formel Ib überführt.

29. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

30. Mittel gemäss Anspruch 29, dadurch gekennzeichnet, dass es als aktive Komponente mindestens eine der in den Ansprüchen 2 bis 27 definierten Verbindungen enthält.

31. Verfahren zur Herstellung eines in den Ansprüchen 29 und 30 definierten (agro)chemischen Mittels, dadurch gekennzeichnet, dass man mindestens eine Verbindung der Formel I gemäss Anspruch 1 mit geeigneten festen oder flüssigen Zusatzstoffen und Tensiden innig vermischt.

32. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 27 auf die Pflanze, Pflanzenteile oder deren Standort appliziert.

33. Verfahren zum Beizen von Saatgut, dadurch gekennzeichnet, dass dieses mit einer Verbindung der Formel I gemäss Anspruch 1 behandelt wird.

34. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss Anspruch 1.